# EUROPEAN PATENT SPECIFICATION

(11) **EP 2 813 233 B1**
(45) Date of publication and mention of the grant of the patent: **15.08.2018**
(21) Application number: 13196952.9
(22) Date of filing: 12.12.2013
(51) Int. Cl.: A61K 36/77, A61K 36/16, A61P 3/10

(54) **COMPOSITION COMPRISING NATURAL EXTRACTS**
ZUSAMMENSETZUNG MIT NATÜRLICHEN EXTRAKTEN
COMPOSITION COMPRENANT DES EXTRAITS NATURELS

(30) Priority: 13.06.2013 IL 22691313; 13.06.2013 US 201313917129
(43) Date of publication of application: 17.12.2014
(73) Proprietor: Inno-Bev Ltd., 6345324 Tel Aviv (IL)
(72) Inventor: Faraggi, Eli, 6424508 Tel Aviv (IL)
(74) Representative: J A Kemp

(56) References cited:
- EP-A1- 1 671 550
- WO-A1-03/101225
- DE-A1-102004 017 512
- US-A1- 2006 134 300
- US-A1- 2006 147 600
- SCHIMPL FLÁVIA CAMILA ET AL: "Guarana: revisiting a highly caffeinated plant from the Amazon.", JOURNAL OF ETHNOPHARMACOLOGY 28 OCT 2013, vol. 150, no. 1, 28 October 2013 (2013-10-28), pages 14-31, XP55108967, ISSN: 1872-7573
- SMITH N ET AL: "Guarana's journey from regional tonic to aphrodisiac and global energy drink", EVIDENCE-BASED COMPLEMENTARY AND ALTERNATIVE MEDICINE 2010 OXFORD UNIVERSITY PRESS GBR, vol. 7, no. 3, September 2010 (2010-09), pages 279-282, XP55108946, ISSN: 1741-427X
- TANAKA S ET AL: "Effects of the flavonoid fraction from Ginkgo biloba extract on the postprandial blood glucose elevation in rats", YAKUGAKU ZASSHI 200409 JP, vol. 124, no. 9, September 2004 (2004-09), pages 605-611, XP55108970, ISSN: 0031-6903
- WOLFGANG SCHINZ: "Fruit sweeteners in vogue.", INTERNATIONAL FOOD INFORMATION SERVICE (IFIS), FRANKFURT-MAIN,DE, vol. 66, no. 4, 1 January 1999 (1999-01-01), pages 174-175, XP8168295, FLUESSIGES OBST 1999 HERBAFOOD NAHRUNGSMITTEL GMBH, D-14542 WERDER, GERMANY
- MATTHIAS SASS: "Functional ingredients with measurable health benefits.", FRUIT PROCESSING, vol. 18, no. 1, 1 January 2008 (2008-01-01), pages 14-16, XP8168305, FRUIT PROCESSING 2008 RUDOLF WILD GMBH & CO KG, D-69214 HEIDELBERG/EPPELHEIM, GERMANY

## Description

### TECHNOLOGICAL FIELD

This invention relates to compositions comprising plants extracts that improve the well being of a subject.

### BACKGROUND

Plants formulations are widely used to provide health benefits. For example plants such as *guarana, ginkgo biloba, ginseng* [sp] and others were previously reported to improve health conditions either when used alone or in combinations with additional ingredients.

For example, *guarana* seeds were described to have stimulant effects which are generally attributed to the presence of caffeine comprising 2.5%-5% of the extract's dry weight [Haskell CF et al., J. Psychopharmacology, 21, 65-70; 2007]. In this publication, *Guarana* was described to exhibit psychoactive properties being attributed to high content of additional possible psychoactive components including saponins and tannins. Further, this publication shows the effect of guarana on mood with dose dependent increase in alertness and contentedness. As described, two low concentrations (37.5 mg and 75 mg extracts) allegedly provided more beneficial cognitive effects as compared to two higher concentrations (150 mg and 300 mg extracts).

Further, it was described that administration of 75 mg of dried ethanolic extract of *guarana* (approx 12% caffeine) to non-fatigue individuals led to improvement in secondary memory and speed of attention. It was hypothesized that given the low caffeine content (9 mg) of this dose of *guarana* extract, the effects were unlikely to be attributable to its caffeine content [Kennedy DO et al., Pharamcol, Biochem Behav, 79: 401-411; 2004].

In some other publications, such as International Application Publication Nos. WO06/065255 and WO03/101225 compositions are describes as comprising a plant extract (for example *guarana,* ginkgo and ginseng) mixed with caffeine and/or taurine and sugars.

### SUMMARY OF DESCRIPTION

The present invention provides a composition comprising
(i) between 30mg to 70 mg of an extract of *guarana* plant or an isolate of *guarana* plant obtained by other isolation methods (not extraction) per 100ml of composition,
(ii) between 30mg to about 70mg of an extract of *ginkgo biloba* plant or an isolate of *ginkgo biloba* plant obtained by other isolation methods (not extraction) per 100ml of composition and
(iii) between 4ml fruit sugars to 15ml fruit sugars per 100ml of composition,
for use in ameliorating or inhibiting the temporary reduction of physical and/or mental functionality of a subject who is experiencing post lunch dip,
wherein the composition is administered at least once a day for a period of at least 7 days, and
wherein the composition exhibits a medically negligible or no detected effect on the subject's heart activity following administration.

In some embodiments, the composition also comprises elderberry. In yet some other embodiments, in 100ml final composition the elderberry is present in an amount of between about 40mg to about 60mg.

Without being bound by theory, it is believed that the *guarana* and *ginkgo biloba* from plants, each contain between 20% to 30%, at times, between 24% to 29% and further at times, about 24% of active ingredients. As such, in some embodiments, in a 100ml composition the following amounts of active components are included: (i) between about 6mg *guaranine* to about 20mg *guaranine* (at times between 7 to 17mg); (ii) between about 6mg to about 20mg *flavonoid glycosides* (at times between 7 to 17mg) ; and (iii) between about 4ml to about 15ml fruit sugars.

In some other embodiments, the subject's heart activity is determined by one or more parameters indicative of a subject's heart activity, said one or more parameters being selected from the group consisting of pulse rate, heart rate and blood pressure.
As a result, the composition is particularly suitable for administration to patients whose heart activity is already compromised in some way. Thus, typically, the composition is administered to a patient suffering from a pre-existing heart condition or a condition that would be aggravated by tachycardia. Such conditions are well known and include, for example, cardiac arrhythmia, hypo- or hypertension, angina or angina-like complaints, history of myocardial infarction, coronary artery disease or elderly patients. Typically, the composition is administered to a patient who is receiving one or more further therapeutic agents which provoke or aggravate tachycardia or hypertension. Therapeutic agents that provoke or aggravate tachycardia are well known and include, for example, adrenaline sympathomimetics such as alpha-agonists and beta-agonists, dopamine sympathomimetics, anticholinergics, antihistamines, amphetamines and theophylline. Therapeutic agents that provoke or aggravate hypertension are well known and include, for example, non-steroidal antiinflammatories, decongestants, migraine medications and weight loss drugs.

The composition is particularly suitable for oral consumption, e.g. in the form of a drink or in a form suitable for forming a drink (e.g. dissolvable or dispersible tablet, gel or capsule) and chewing gum and gummy bears.

The composition may be provided at any time either during or before post lunch dip is developed. Alternatively, the composition may be administered before noon or within a time window of up to an hour before post lunch is developed in a subject in need thereof.

### BRIEF DESCRIPTION OF THE DRAWINGS

In order to understand the disclosure and to see how it may be carried out in practice, embodiments will now be described, by way of non-limiting example only, with reference to the accompanying drawings, in which:
**Figures 1A** and **1B** are bar graphs showing the effects of *composition 1.*
**Figures 2A-2E** are bar graphs showing the effects of *composition 2 ("wake up")* on the vigilance rating (VAS, **Figure 2A****),** on the focus ability **(****Figure 2B****),** on the effectiveness at work **(****Figure 2C****),** on the immediate correct words recall test **(****Figure 2D****)** and on the number of correct symbols in the digit symbol substitution test (DSST, **Figure 2E****).**
**Figures 3A-3D** are bar graphs showing the effect of *composition 3* on the correct symbols in the DSST (**Figure 3A**), on the vigilance **(****Figure 3B****),** on the focus ability **(****Figure 3C****),** and on the immediate word recall test **(****Figure 3D****).**
**Figures 4A-4D** are bar graphs showing the effect of daily dosing of *composition 3* on the immediate word recall test **(****Figure 4A****),** on the correct symbols in the DSST **(****Figure 4B****),** on the vigilance **(****Figure 4C****),** and on the work effectiveness - VAS scores **(****Figure 4D****).**

### DETAILED DESCRIPTION OF EMBODIMENTS

Energy drinks often include high amounts of (above 50mg/100ml) stimulates such as caffeine and/or taurine. Drinking coffee is also known to have waking effect. However, caffeine has a short half-life and potential side effects, such as increased pulse rate and blood pressure. In addition, regular coffee drinking, at times, results in tolerance and substantial reduction of the effect of caffeine. When referring to tolerance (physiological tolerance) it is to be understood as referring to a decrease in the response of a subject to the compositions disclosed herein, due to previous exposures to the composition.

The present disclosure provides alternative awakening compositions. In the broadest aspect, the present disclosure provides a composition for improving a subject's vigilance or performance without the negative side-effects of energy drinks currently on the market.

Specifically, the inventors developed compositions that lead to significantly improvement in cognitive capabilities and awakeness parameters measured after lunch time, without increasing the subject's blood pressure and heart rate to an extent typically occurring after drinking coffee or energy drinks having high amounts of caffeine, taurine or the like. In addition, the inventors found that when the developed composition was used on a daily basis for a period of several days no tolerance to the composition's effect was observed.

Further, the inventors compared the effect following drinking of a composition disclosed herein to the effect of drinking caffeine and observed that the composition disclosed herein was at least as good as caffeine in its capability to improve awakeness and mental capabilities. However, while caffeine consumption resulted in an increase in blood pressure and heart rate, the composition disclosed herein was advantageous as it was not associated with any similar increase in heart activity.

In addition, use of the composition disclosed herein, on a daily basis, for a period of several days, resulted in a gradual improvement of cognitive measures (e.g. accumulated effect). In other words, the inventors observed a gradual increase in the measured parameters over time of the study.

When referring to a **"*plant*"** it is to be understood to encompass the plant as a whole, as well as one or more parts thereof, including leaves, stems, seeds, flowers, fruits and roots. The plant may be subject to extraction as a fresh plant (e.g. as a whole plant) or the plant may be a priori processed by any one of drying, semi-drying, cutting, chopping, grinding, powdering and the like.

When referring to a **"*isolate*"** it is to be understood as encompassing a natural substance obtained from a plant as well as any synthetic or semi-synthetic analog thereof having a biological activity corresponding to the activity of the natural substance (the natural, synthetic or semi-synthetic isolate being regarded as active ingredient, active material, active component of the plant). In the context of the present disclosure, the isolate is obtained from the plant by other isolation methods (not by extraction techniques).

When referring to an **"*extract"*** it is to be understood as encompassing a product of separation (or isolation) of one or more substances from a plant or a plant part using solvents. The extract is typically obtained with a water-solvent system. Plant extracts are well known in the art and may use a variety of solvents, such as, without being limited thereto, pentane, decane, cyclohexane, hexane, petroleum ether, monochloromethane, ethanol, butanol, acetone, dichloromethane, chloroform, isopropanol, propanol, ethyl acetate, methanol, butylene glycol, propylene glycol, pentylene glycol, glycerol, ethers, oils, supercritical carbon dioxide, water or any other suitable solvent, and any mixture of these solvents.

In one embodiment, the plant extract is obtained in a water-alcohol system, such as, without being limited thereto, water-ethanol system.

As noted above, each isolate (for example in the form of an extract) comprises measurable **"*active substances*",** believed to be the substances or components of the extract that are responsible for the awaking effect of the final composition (namely, wakefulness). In this context it is to be appreciated that only a portion of the isolate from a plant constitutes the active substances and thus, for instance, 50mg of an extract may include only a small fraction of active substances. The amount of active substances in an isolate may be determined by analytical methods, such as High Performance Liquid Chromatography (HPLC), chromatography, spectroscopy and the like.
When referring to **"*awaking effect*"** it is to be understood as encompassing any effect improves the physical and/or mental functionality of a subject. Without being limited thereto, the term **"*awaking effect*"** denotes improvement in mental capabilities such as anyone of vigilance, immediate word recall, decision making and speed of response, short memory, concentration, focus, effectiveness, increased performance, or similarly reduction in anyone of sleepiness or fatigue.

In some embodiments, the awaking effect is desirable for subject experiencing temporary reduction in the above mentioned physical and/or mental functionalities, e.g. when the subject is experiencing post lunch dip (PLD).

PLD is a phenomena associated with deterioration of function and productivity typically between noontime and 16:00. PLD is sometimes regarded as a circadian phenomenon, related with circadian rhythm of the body since it was reported to occur regardless of lunch or food intake. However, its effect may be more pronounced following lunch. PLD may be also attributed to various reasons, including, for example, hormonal (which can also be regulated by a circadian pacemaker) or nutritional/gastrointestinal changes. Another explanation for PLD may be related to body temperature changes (a decrease in the body temperature typically occurring in early afternoon hours which increases the tendency to sleep), decreased cortisone/cortisol levels, and re-distribution of blood leading to sleepiness, particularly during noon - early afternoon hours.

The term *"ameliorating, reducing* or *inhibiting"* as used herein, refers a complete range of positive effects of administrating to a subject a composition as disclosed herein.

By *"subject"* it is meant a human (at times considered patient) who may be affected by the conditions detailed herein, for example post lunch dip and the associated conditions and to whom the composition or method described herein is desired. In some embodiments, the subject being administered with the composition described herein is considered to exhibit a response to the composition.

The term *"response"* or *"responsiveness"* refers to an improvement in at least one relevant parameter after being administered with the composition as compared to
subject not being administered with the composition or as compared to the parameters of the same subject prior to administration.

The parameters of the same subject may be determined prior to administration of the composition or at any other time provided that the subject has not being administered with the composition.

In some embodiments, the parameters are associated with the *"**awaking effect"*** as described herein. For example, the parameters may be an overall feeling of the subject for example an overall awaken feeling, fewer tendencies to sleep, vital feeling.

In some other embodiments, the parameters validated used function and vigilance tests. In some further embodiments, the parameters are psychomotor, cognitive or behavioral tests. In yet some further embodiments, the measured parameter is at least one of immediate word recall test (short term memory), digit symbol substitution test (concentration), and subjective rating (on a visual analogue scale - VAS) of their vigilance, ability to focus, and effectiveness at work.

As appreciated:
**VAS** refers to a visual-analog test in which the participant described on a line of 10cm scale his/her subjective feeling regarding the questioned parameter. The titles in the 3 scales which were used were: somnolent - alert; confused - focused; and none-effective - effective in performance/work. Subjects were asked to cross the line according to their state at each test and the score was calculated as the length (in cm) from the left side of the line till the point that they marked. Thus, the numbers run from 0 to 10, with higher scores indicating higher subjective alertness or better performance. These scales are widely used to assess subjective complaints including somnolence, although do have some limitations.
**DSST** refers to the Digit Symbol Substitution Test is a time limited test in which a patient is required to replace digits by symbols in a given time restriction (2 min). The test provides data on the accuracy and rate of performing the task, and is a very common tool to assess function and compare between various sleep/alert conditions.
**iWRT** refers to the immediate Word Recall Test is a test of short term memory which is a very common tool to assess cognitive function and compare between various sleep/alert conditions. Thirty unrelated words were presented to the participant, each word for 2 seconds, and at the end the participant was required to recall as many words as s/he could. Both correct and incorrect or repeated words were analyzed. It has been shown to reliably quantify short term memory as a function of the frontal lobe, which can be impaired by sleepiness and improved by alertness

In the context of the present disclosure an improvement in at least one of the measured parameters relates to providing an awaking effect and/or inhibiting or reducing post lunch dip. A measured improvement is defined as an improvement of at least 3%, at times at least 5%, at times at least 20%, at times even at least 50%. The measured improvement is defined as a statistically significant improvement as determined by conventional statistical tests (such as t-test).

Further in the context of the present disclosure an improvement in at least one of the parameters (including overall feeling) is considered as an improvement or response to the composition.

As detailed herein below, the composition may be administered on a need by a subject or may be administered on a daily basis.

The composition disclosed herein was found to have minimal (analysis of variance with p<0.05 considered statistically significant), if at all, effect on the subject's heart activity, this being compared, at least to the effect to 55mg caffeine.

Thus, in accordance with some embodiments, the composition disclosed herein exhibits an advantageous medically negligible or no detected effect on the subject's heart activity following administration thereof to the subject.

***Heart activity*** as used herein includes any one of the following parameters: heart rate, blood pressure, (systolic and diastolic blood pressure), etc.

When referring to a ***"medically negligible"*** or ***"no detected effect"*** it should be understood as referring to a marginal change which is statistically not significant as determined by conventional statistic tests (such as t-test) or to an effect which is below the changes that can be detected using conventional methods. In some embodiments, the heart activity is determined before consumption of the composition and compared to the activity determined after consumption of the composition in order to show the marginal or no effect on the heart activity.

In some embodiments, the heart activity is determined within a time window of up to 24 hours post administration of said composition, at times up to 12 hours post administration and at times up to 6 hours post administration.

Use of the composition of the invention involves administration of an effective amount of the composition to a subject.

As used herein the term *"effective amount"* is intended to mean that amount of a composition, and in particular of the active components therein, that will improve the condition of the subject and in the context of the present disclosure provide ***"awaking effect"*** as described herein. In some embodiments this term refers to an amount of the composition and in particular of the active components therein, which is administered to a subject in need thereof, necessary to effect a beneficial change in the condition or to reduce, inhibit or ameliorate the condition or development of the condition. In some other embodiments, the amount to be administered *ameliorates, reduces* or *inhibits* for example post lunch dip without affecting the subject's heart activity.

In some embodiments, providing or administering the composition described herein induces a positive response to the composition (or to method of administrating the composition).

In the context of the present disclosure, when referring to *guarana* it is to be understood as meaning Paullinia cupana also known by the names Paullinia crysan, and Paullinia sorbilis.

In accordance with the present disclosure, the isolate of *guarana* used herein, is prepared by extraction of any plant part thereof. In some embodiments, the extract is of *guarana* seeds. Seed extraction may be obtained by several methods known in the art. For example, seed extraction is carried out in an aqueous ethanol system after which the ethanol is evaporated and the residual *guarana* extract is dried. The dry extract may be hydrated prior to use. The seeds may be fresh or dry before extraction.

In some embodiments, the *guarana* extract is *guarana* seed extracts.

The amount of *guarana* extract in the composition may vary. In some embodiments, the composition comprises between about 30mg *guarana* extract /100ml composition to about 70mg *guarana* extract /100ml composition.

In some other embodiments, the composition comprises between about 40mg *guarana* extract/100ml composition to about 60mg *guarana* extract/100ml composition. These amounts may correspond to about 0.04% to about 0.06% (w/v) of *guarana* extract in the final composition.

In some embodiments, 100 ml of the composition comprises between about 45 mg to 55 mg of *guarana* extract, at times, between about 48mg to 51mg of *guarana* extract.

In one embodiment, 100 ml of the composition comprises about 49 mg of *guarana* extract.

As appreciated, the extract comprises active substances some of which are known in the art. For example, *guarana* and this includes *guarana* seeds are known to include *guaranine* (caffeine), tannins and saponins. In addition, *guarana* may include for example alkaloids such as theophylline and theobromine.

In the context of the present disclosure the active substances in *guarana* include without being limited herein to *guaranine* (caffeine), tannins, saponins and alkaloids such as theophylline and theobromine.

When referring to *guaranine* it should be understood to include also the other active substances disclosed herein.

As noted above, the final composition comprises between 30 to 70 mg of an extract of *guarana* plant or an isolate of *guarana* plant obtained by other isolation methods (not extraction). Such amount of *guarana* isolate from a plant provides, in some embodiments, between about 6mg to about 20mg *guaranine* in 100ml of the final composition, at times between 7 to 17mg *guaranine* in 100ml of the final composition.

In some other embodiments, the *guarana* extract provides about 14mg guaranine in 100 ml of final composition. This amount may be regarded as constituting 20% to 30%w/v of guaranine (in 100ml final composition), at times between 24% to 29% or about 24% of guaranine (in 100ml final composition).

In some further embodiments, the *guarana* isolate used here refers to *guaranine* prepared by synthetic methods or by modification of naturally obtained *guaranine* (e.g. semi-synthetic).

In some embodiments, the isolate of *ginkgo biloba* used herein, is prepared by extraction of any plant part thereof. In some embodiments, the extract is of *ginkgo biloba* leaves.

As noted above, extraction may be obtained by several methods known in the art. For example, *ginkgo biloba* leaves extract may be obtained using an ethanol/water extraction system, wherein the desired fraction of the extraction (the *ginkgo biloba extract)* is within the water fraction.

In some embodiments, the *ginkgo biloba* extract is *ginkgo biloba* leaves extract.

The amount of *ginkgo biloba* extract in the composition may vary. In some embodiments, the composition comprises between about 30mg *ginkgo biloba* extract /100ml composition to about 70mg *ginkgo biloba* extract /100ml composition

In some other embodiments, the composition comprises between about 40mg *ginkgo biloba* extract /100ml composition to about 60mg *ginkgo biloba* extract /100ml composition. These amounts may correspond to about 0.04% to about 0.06% (w/v) of *ginkgo biloba* extract.

In some embodiments, 100 ml of the composition comprises between about 45 mg to 55 mg of *ginkgo biloba* extract, at times, between about 48mg to 51mg of *ginkgo biloba* extract.

In one embodiment, 100 ml of the composition comprises about 49 mg of *ginkgo biloba* extract.

As appreciated, the extract comprises active substances some of which are known in the art. For example, *ginkgo biloba* and this includes *ginkgo biloba* leaves are known to include flavonoid glycosides and terpenoids (ginkgolides, bilobalides). In accordance with one embodiment, the amount of flavonoid glycosides is determined as the amount of active substances in the *ginkgo biloba* extract. The amount may be determined using, for example, HPLC.

As noted above, the final composition comprises between 30 to 70 mg of an extract of *ginkgo biloba* plant or an isolate of *ginkgo biloba* plant obtained by other isolation methods (not extraction). Such amount of *ginkgo biloba* extract provides, in some embodiments, between about 6mg to about 20mg flavonoid glycosides in 100ml of the final composition, at times between 7 to 17mg flavonoid glycosides in 100ml of the final composition.

In some other embodiments, the *ginkgo biloba* extract provides about 11mg flavonoid glycosides in 100 ml of final composition. This amount may be regarded as constituting between 20% w/v to 30%, at times between 24% to 29% or about 24% of flavonoid glycosides (in 100ml final composition).

As appreciated, various flavonoid glycosides may be found in plants and these include, without being limited thereto, hesperidin, naringin, rutin and quercitrin.

In some other embodiments, the isolate of *ginkgo biloba* comprises synthetic flavonoid glycosides.

In some embodiments, the composition comprises in 100ml final composition between about 6mg to about 20mg *guaranine* and between about 6mg to about 20mg *flavonoid glycosides.*

The composition of the present disclosure may comprise for example *guarana* isolate from plant and synthetic flavonoid glycosides or visa versa, namely synthetic *guaranine* and flavonoid glycosides isolate from plant.

In some embodiments, in a 100ml composition the following amounts are included: (i) between about 6mg *guaranine* to about 20mg *guaranine* (at times between 7 to 17mg); (ii) between about 6mg to about 20mg *flavonoid glycosides* (at times between 7 to 17mg) ; and (iii) between about 4ml to about 15ml fruit sugars.

While it is known that *ginkgo biloba* provides nutritional support for mental alertness, short and long term memory, increase reaction time, improved mental clarity, enhance vitality level, improve circulatory health and blood vessel health, its combination with *guarana* at the amounts provided herein, and in the absence of high
amounts of caffeine or other acceptable stimulants (amount above 50mg / 100 ml), exhibited superiority over compositions comprising such amounts of stimulants. In the absence of high amounts of caffeine or the like, the compositions disclosed herein are regarded as being ***"essentially free of stimulant***"*.* The term ***"essentially free of stimulant"*** is used herein to denote that the amount of stimulants, such as caffeine and/or taurine in the composition is not more than 10% w/v of the final composition (out of 100ml final composition).

When referring to "***stimulants***"*,* it is to be understood as denoting any substance known to improve mental or physical function of a subject as described above. Non-limiting examples of stimulants include, without being limited thereto are caffeine and taurine, Vitamin B Complex.

Caffeine is the most common stimulant found naturally in coffee, tea and is widely used in soft drinks specifically in larger amount in energy drinks. It is known to be useful as a cardiac stimulant and a mental stimulant. One drawback in using caffeine resides in its effect in increasing heart pulse rate and blood pressure. In addition, regular coffee drinking results in tolerance and substantial reduction of the effect of caffeine. Taurine is considered as a similar stimulant.

Also included in the final composition disclosed herein are fruit sugars. These include natural fruit sweetener extracted entirely from fruits. In some embodiments, the fruit sugars are selected as those having a low glycemic index.

In some embodiments, the natural fruit sweetener is extracted from apples, grapes, pear, pineapple, orange, peach etc. Such extracts are known to have a Brix level of between 60°Bx to 80°Bx (at 20°C). The fruit sugars may be added to the composition in the form of crystal sugar or sugar syrups. This may be obtained by adding to the final composition an amount constituting between about 4% to about 15% fruit sugar. In some other embodiment, the composition comprises between about 6% to about 10% fruit sugars. In some further embodiments, the composition comprises between about 7% to about 8% fruit sugars (corresponding to between 6 to 10ml fruit sugars in 100ml final composition).

In some embodiments, the fruit sugars are commercially available, such as the commercial all natural fruit sweetener product known as Fruit-up® (WILD Valencia S.A).

In some embodiments, the composition may also comprise additional plant derives substances.

In one embodiment, the composition comprises an extract of elderberry, in particular, the berry of the black elder tree (*Sambucus nigra*) and this provides a source of vitamins A, B and C, bioflavonoids and anthocyanins. Extraction from the berry may be performed by any known technique. In accordance with some embodiments, the extraction is performed using an ethanol/water extraction system. The extract is the fraction soluble in water.

According to some embodiments, elderberry extract is included in the final composition in an amount between about 0.04% to about 0.06% (v/w).

In some embodiments, the elderberry extract was added in an amount between about 0.04% to about 0.05% (v/w).

In some embodiments, the final composition comprises between 40 mg to 60 mg of elderberry extract (in 100ml final composition), at times, in an amount of about 49 mg of elderberry extract in 100 ml final composition.

In some embodiments, the active substance in the elderberry comprises anthocyanins and the amount of anthocyanins in the elderberry extract may be between 1% to 5%, at times between 2% to 4%. In some embodiments, the elderberry extract comprises 3% of anthocyanins. Anthocyanins are found in different plant parts such as leaves, stems, roots, flowers and fruits. Anthocyanins consist of a backbone known as anthocyanidin which is bound to one of three sugars: arabinose, glucose, or galactose.

The composition may comprise additional ingredients such as one or more of artificial sweeteners, minerals, vitamins (such as Vitamin A, Vitamin B1, Vitamin B2, Vitamin B6, Vitamin B12, Vitamin C, Vitamin D3, Vitamin E, Folic Acid). The composition may comprise flavoring agents such as these that may provide for example a lemon taste.

The composition disclosed herein was found to be superior over consumption of 50mg caffeine. While the composition and consumption of 50mg caffeine may be regarded as providing a similar awaking effect, it is clearly exhibited herein (in the following non-limiting examples) that caffeine affected heart rate and blood pressure to an extent that was not observed by the composition disclosed herein.

The composition disclosed herein was found to be suitable for daily dose, as the subjects being given the composition on a daily basis did not develop tolerance to the composition and at times showed even a favorably accumulating effect (e.g. beneficial awaking effect) of the composition.

Specifically as shown in Example 5 herein below, consumption of the composition for a time period of 30 days resulted in an improvement in the validated used function and vigilance tests as detailed herein.

In addition, consumption of the composition for 30 days led to an improvement in the baseline measured. Without being bound by theory it was suggested that the improvement may be attributed to an accumulative effect of the composition.

Interestingly and in contrast to caffeine consumption, the composition was well tolerated for the tested time period.

In some other embodiments, the composition is for use during a period of at least fourteen days. In some further embodiments, the composition is for use during a period of at least thirty days.

In some other embodiments, the composition is for use once or twice a day. In some further embodiments, the composition is for use once a day.

Consumption (use, administration, providing) of the composition of the developed composition may be in general at any time of the day. In some embodiments, the composition is for use during or before post lunch dip is developed. In some other embodiments, the composition is for use before noon or within a time window of up to an hour before post lunch is developed in a subject in need thereof.

In some embodiments, the composition disclosed herein may be provided as a drink, such as an energy drink. For example, the energy drink may be used prior to lunch and may assist in post lunch dip. In some other embodiments, the composition may be used as a beverage. In some other embodiments, the composition may be mixed with other liquids, such as water, flavoring materials, natural juices, etc.

In some other embodiments, the composition may be provided in tablet form, wherein the ingredients are compressed together with microcrystalline cellulose into a tablet. The tablet may be in a form suitable for dissolution in water or any other liquid suitable for human consumption.

### DETAILED DESCRIPTION ON SOME NON-LIMITING EXAMPLES

### Example 1 Preparation of various compositions

### Methods:

The ingredients were mixed where the fruit sugars (polysaccharides) are first dissolved by mixing with water at 25-35°C for at least 20minutes. Then, remainder of the components are added while mixing for an additional period of 30min. Alternatively, sorbate, plants extracts and stabilizers are mixed in 10% of the water prior to the addition of the sugars. At times, mixing is under high sheer forces.

### Results:

The following compositions were formulated:

**Table 1 - Components of Composition 1**

| Ingredient | Amount in 100 ml | |
|---|---|---|
| | liquid (ml) | powder (g) |
| Water (Superherb, Israel) | 82 | |
| Fruit up (Wild) | 8 | |
| Pectin (HF 102) (Shavit , Israel) | | 0.25 |
| Xantham Gum (Shavit , Israel) | | 0.02 |
| Apple concentrate (Black & Decker) | | 10 |
| Vitamin C (Shavit, Israel) | | 0.03 |
| Apple flavor (Wild) | 0.08 | |
| Berries flavor (Wild) | 0.09 | |
| Black carrot (Wild) | 0.024 | |
| Citric acid (anhydrous) (Shavit, Israel) | | 0.1 |
| Sorbate (Shavit, Israel) | | 0.2 |
| Guarana extract (FIC NatuRx) | | 0.063 |
| Elderberry extract (FIC NatuRx) | | 0.05 |
| Ginkgo extract (FIC NatuRx) | | 0.05 |

**Table 2 -Components of composition 2**

| Ingredient | % in 100 ml |
|---|---|
| Water (Superherb, Israel) | 88.397 |
| Fruit up sugar (WILD) | 7.887 |
| Liquid Sucralose (Black & Decker) | 1.183 |
| Grapefruit Concentrate (Black & Decker) | 0.986 |
| Citric Acid (Shavit , Israel) | 0.493 |
| Powder Natural Orange Flavour (WILD) | 0.394 |
| Potassium Sorbate (Shavit, Israel) | 0.197 |
| Liquid Natural Lime flavor (WILD) | 0.197 |
| Liquid Natural Grapefruit flavor (WILD) | 0.089 |
| Elderberry Extract (FIC NatuRx) | 0.049 |
| Guarana Extract (FIC NatuRx) | 0.049 |
| Ginkgo Extract (FIC NatuRx) | 0.049 |

**Table 3-Components of composition 3 ("Wakeup")**

| Ingredient | % in 100 ml |
|---|---|
| Water (Frutarom, Israel) | 87.5 |
| Fruit up sugar (Wild) | 7.9 |
| Sugar (Frutarom, Israel) | 4.0 |
| Sucralose (Frutarom, Israel) | 0.0075 |
| Citric Acid (Frutarom, Israel) | 0.35 |
| Potassium Sorbate (Frutarom, Israel) | 0.02 |
| Xantham Gum (Frutarom, Israel) | 0.03 |
| Elderberry Extract (FIC NatuRx) | 0.049 |
| Guarana Extract (FIC NatuRx) | 0.049 |
| Ginkgo Biloba Leaf Extract (FIC NatuRx) | 0.049 |
| Masking Nat (Frutarom, Israel) | 0.030 |
| Lemon Grass (Frutarom, Israel) | 0.02 |

**Table 4 -Components of composition 4 (caffeine composition)**

| Ingredient | Amount in 100 ml | |
|---|---|---|
| | Liquid (ml) | Powder (gram) |
| water | 82 | |
| Glucose | 8 | |
| Pectin | | 0.25 |
| Xantham Gum (Shavit, Israel) | | 0.02 |
| Apple concentrate (Black & Decker) | | 10 |
| Apple flavor (Wild) | 0.08 | |
| Berries flavor (Wild) | 0.09 | |
| Black carrot (Wild) | 0.024 | |
| Citric acid (anhydrous) | | 0.1 |
| Sorbate (Shavit, Israel) | | 0.2 |
| Caffeine (Superherb, Israel) | | 0.050 |

**Table 5 -Components of composition 5 (Placebo)**

| Ingredient | Amount in 100 ml | |
|---|---|---|
| | Liquid (ml) | Powder (gram) |
| water | 82 | |
| Glucose | 8 | |
| Pectin | | 0.25 |
| Xantham Gum (Shavit , Israel) | | 0.02 |
| Apple concentrate (Black & Decker) | | 10 |
| Apple flavor (Wild) | 0.08 | |
| Berries flavor (Wild) | 0.09 | |
| Black carrot (Wild) | 0.024 | |
| Citric acid (anhydrous) | | 0.1 |
| Sorbate (Shavit, Israel) | | 0.2 |

### Example 2 - Sensory and consumer study

### Method:

The study population included 98 adults (ages 25 to 35), 60% men and 40% women with 68% out of the total population having an academic degree.

Each participant was provide with 4 drinks of *composition 1* and was requested to drink one drink after lunch for a time period of 4 days and a list of questions. Participants' remarks on questions were monitored after 4 days of drinking the formulation.

### Results:

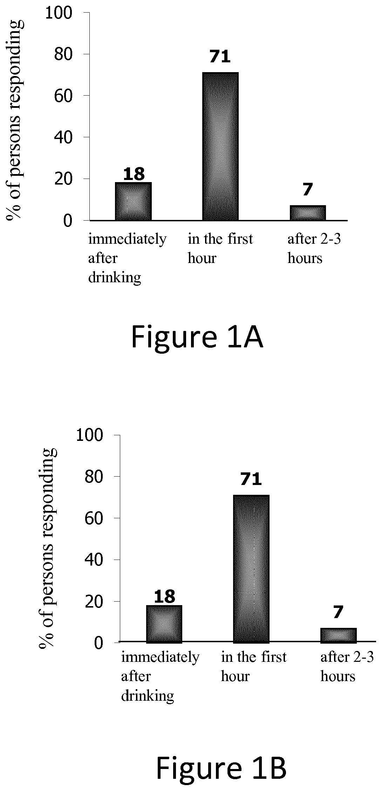

The results of this study obtained with *composition 1* indicated that drinking the formula after lunch, 71% of the participants reported that they felt awake one hour after drinking the formula (**Figure 1A**). 66% reported that awakeness was preserved for a time period of 2-3 hours (**Figure 1B**).

### Example 3 Clinical studies

### Methods:

A group of thirty healthy non smoker volunteers (13 male, 17 female) was recruited to the test via advertisements in the faculty of medicine. The volunteers had an average age of 36.6±12.4 years (range 18-61) and an average BMI value of 24.3±3.5 Kg/m2 (range 17.0-31.8). They were all in stable medical condition, free of chronic diseases or medications.

In the first study visit they were given explanations on the study, signed the consent form, and were trained with the performance tests. Thereafter, the study consisted of 3 additional visits, with 6±3 days between each 2 study visits. All 3 study visits were performed in a similar fashion, on identical time of the day, as described below.

In each visit volunteers had a standard lunch between 12:00 - 13:00, including for example meat, fish, rice, vegetables, or a vegetarian dish with no desert. After lunch, each volunteer drank either *composition 2* (tested formula), 50 mg caffeine (*composition 4,* Caffeine control composition) or placebo (*composition 5)* in a cross over double blind regimen. All three drinks had a similar look and taste, and were in a similar bottle containing 100ml volume.

Volunteers were asked to maintain a stable and regular schedule, and maintain a stable sleep/wake regimen during the study period. They were asked to maintain a stable time in bed and daytime activity during the period of the study. Specifically they were asked to go to bed and wake up in the morning at the same times before and in the days of the study visits. They were also asked to maintain a similar breakfast in all three mornings of study visits.

Each visit had the same routine: participants had a lunch and immediately after it they drank 100ml of *composition 2, composition 4* (caffeine) or *composition 5* (placebo).

Each volunteer was tested 30 min and 120 min following drinking, by measuring vital signs, blood pressure, and validated commonly used standard function and vigilance tests such as an immediate word recall test (short term memory), digit symbol substitution test (concentration), and subjective rating (on a visual analogue scale - VAS) of their vigilance, ability to focus, and effectiveness at work.

The results of the three visits were compared utilizing one way analysis of variance, with p<0.05 considered statistically significant.

This study was approved by the institutional review board (IRB, Helsinki committee) of Rambam Medical Center and all participants have signed an informed consent prior to participation.

### Psychomotor/cognitive and behavioral tests utilized in the study:

**VAS** - a visual-analog test in which the participant described on a line of 10cm scale his/her subjective feeling regarding the questioned parameter. The titles in the 3 scales which were used were: somnolent - alert; confused - focused; and none-effective - effective in performance/work. Subjects were asked to cross the line according to their state at each test and the score was calculated as the length (in cm) from the left side of the line till the point that they marked. Thus, the numbers run from
0 to 10, with higher scores indicating higher subjective alertness or better performance. These scales are widely used to assess subjective complaints including somnolence, although do have some limitations.

**DSST** - the Digit Symbol Substitution Test is a time limited test in which a patient is required to replace digits by symbols in a given time restriction (2 min). The test provides data on the accuracy and rate of performing the task, and is a very common tool to assess function and compare between various sleep/alert conditions.

**iWRT** - immediate Word Recall Test is a test of short term memory which is a very common tool to assess cognitive function and compare between various sleep/alert conditions. Thirty unrelated words were presented to the participant, each word for 2 seconds and at the end the participant was required to recall as many words as s/he could. Both correct and incorrect or repeated words were analyzed. It has been shown to reliably quantify short term memory as a function of the frontal lobe, which can be impaired by sleepiness and improved by alertness.

### Results:

All volunteers completed the study. Two participants suffered from dizziness after drinking the composition containing 50mg caffeine. No side effects or complaints have observed following drinking *compositions 2* or *5,* namely the tested formula and the placebo.

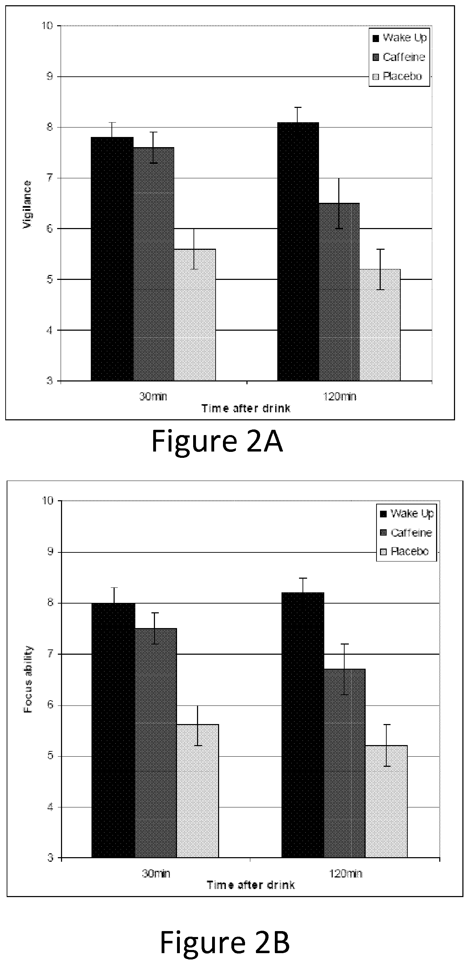

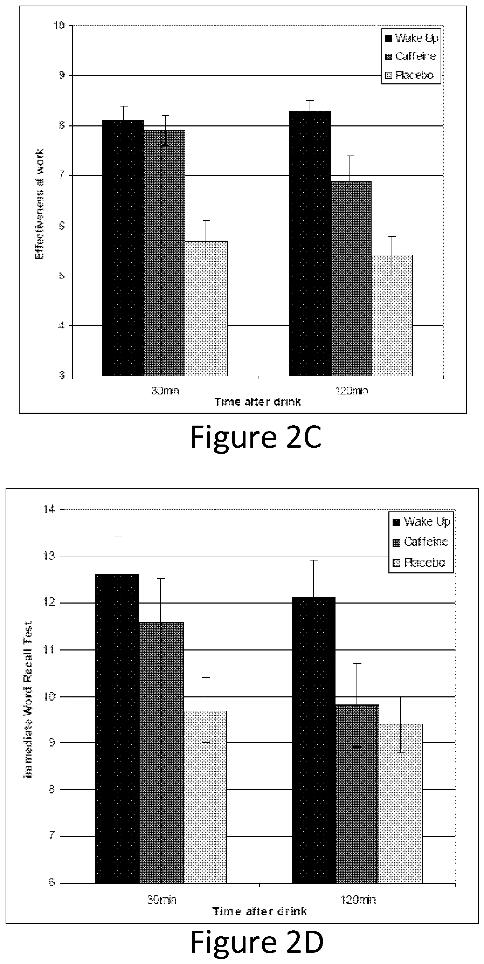

In all performance tests and subjective vigilance and effectiveness assessment, the results obtained after drinking both *composition 2* or *5* (tested composition and caffeine-control composition) were significantly better 30 min following lunch compared with placebo. However, 2 hours following lunch, the effect of the tested formulation was maintained while the effect caffeine containing drink was deteriorated (**Figures 2A, 2B** and **2C**).

As can be seen, 30 min after drinking *compositions 2* and *5* there was a significant improvement. However, 2 hours following drink there was a deterioration of the self rating following caffeine (in all 3 dimensions), and only following "Wake up" vigilance and performance remained high.

As can be seen in **Figure 2D****,** the number of correct words recalled 30 min after drinking was somewhat similar between the group drinking the formula and the ones drinking caffeine (12.6±4.1 and 11.6±4.8, respectively) significantly better (p<0.05) compared with the group drinking the placebo drink (9.7±3.8).

However, 2 hours following the drinking, the number of words recalled was significantly higher (12.1±4.3) in the group drinking formula compared with the groups drinking either caffeine or placebo (9.8±4.9 and 9.4±3.5, respectively) (**Figure 2D**).

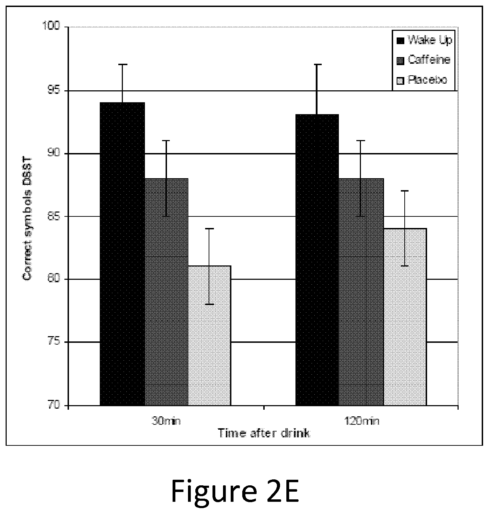

**Figure 2E** shows that the number of correct symbols 30 min and 120 min after drinking was obtained in the group drinking the tested formula both after 30 min and 120 min after drinking.

The pulse rate and blood pressure, measured 30 min after drinking, were significantly higher following drinking caffeine compared to the tested formula: 77.4±1.9/min *vs*. 71.9±1.8/min and 119/75 *vs*. 113/71mmHg, p<0.05. Two hours after drinking the values of these two parameters were similar in the three groups.

These results indicate that drinking the tested formula after lunch improves vigilance and performance 30 min after drinking, same as caffeine and significantly better than placebo. When tested 120 min after drinking, performance and vigilance in the group drinking the tested formula remains significantly higher than both placebo and caffeine. While Caffeine was associated with increasing pulse and blood pressure 30 min after drinking, in the group drinking the tested formulas no hemodynamic differences were observed compared with placebo, both 30 min and 120 min after drinking.

### Example 4 - Clinical studies

### Methods:

A group of 20 healthy volunteers (10 male, 10 female) was recruited to the test. The volunteers had an average age of 37±11 years (range 19 to 63), an average height of 170 cm and average weight of 73 Kg.

Lunch was provided between 12:00 to 13:00 and immediately thereafter the volunteers were examined (time "0"). The volunteers then drank *composition 3* ("*Wakeup"* composition) and were examined again 30 min and 120 min after drinking.

Each volunteer was tested by measuring pulse, blood pressure, and validated commonly used standard function and vigilance tests such as an immediate word recall test (short term memory, IWRT), digit symbol substitution test (concentration, DSST), and subjective rating (on a visual analogue scale - VAS) of their vigilance, ability to focus, and effectiveness at work.

### Results:

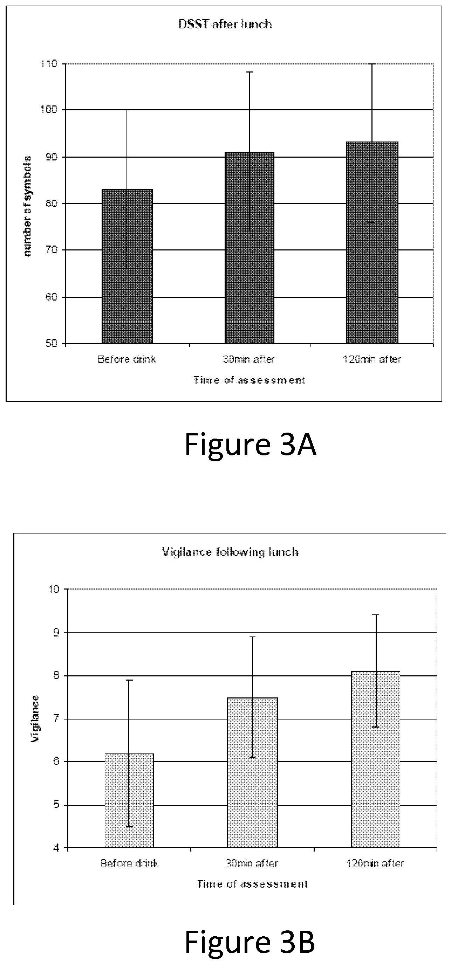

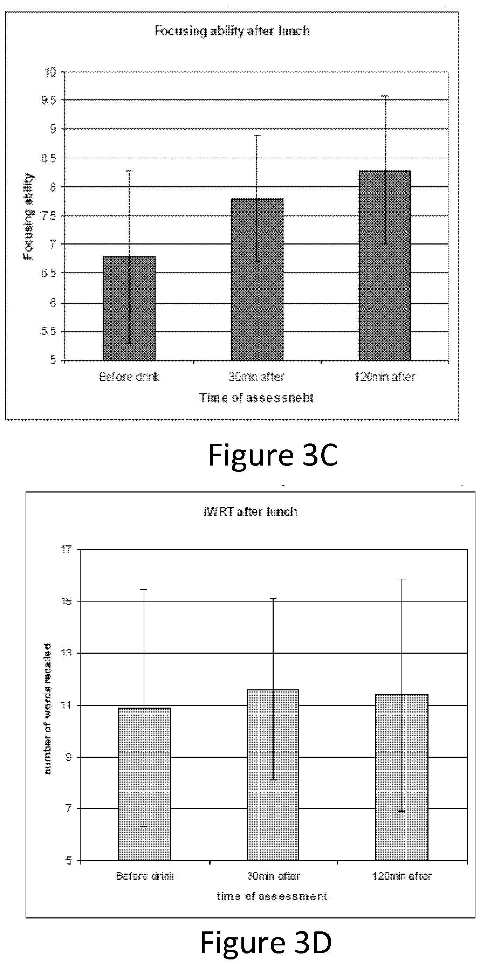

A statistically significant improvement in the capability of switching numbers of symbols (**Figure 3A**), in the vigilance (**Figure 3B**) and on the focus ability (**Figure 3C**) was observed 3o min and 120 min after drinking the composition. In addition, the number of correct words recalled 30 min after drinking was also enhanced after drinking the composition (**Figure 3D**).

The pulse rate and blood pressure, measured 30 min and 120 minutes after drinking were almost unaffected by drinking the composition.

Therefore, based on these results it appears that the tested composition (*Wakeup composition 3*) is effective in counteract the somnolence and reduced performance during the post lunch hours.

### Example 5 -Clinical studies - Long Term Administration

As shown above, individual who drank *composition 3* were observed with a significant improvement in the capability of switching numbers of symbols, in the vigilance and in the focus ability, without any observed increase in the blood pressure and pulse rate.

The objectives of this study were to evaluate the effects of *composition 3* on individuals who were provided with this composition on a daily basis for a month.

### Methods:

A group of ninety five healthy volunteers above 18 years old were recruited to the test.

Measurements were conducted at three different time points: the first measurement was on the first day of the study that included drinking *composition 3* (*Wakeup*) ("*day 1*"), the second measurement was on the last day of the study, namely day 30 of the study ("*day 30*"), and the third measurement was on the next day after the second measurement, namely in the first day without drinking after the 30 days of drinking ("*day 31*").

On day 1 and day 30 of the study the volunteers reported to the hospital at 11:45 (before noon) and had a standard lunch as detailed above.

Immediately after lunch on both measurement days, the volunteers underwent a battery of functional tests and hemodynamic measurements (as detailed below), and then drank one bottle of *composition 3* (100cc). After an hour (at about 13:00) the volunteers underwent a repeated battery of the same tests.

Volunteers continued to drink *composition 3* on daily basis immediately after lunch for a month. Namely, a total of 29 days between the first measurement (*day 1*) and the second measurement (*day 30*). Thus, volunteers drank *composition 3* without reporting to the hospital between day 2 and day 29 of the study and overall drank *composition 3* for 30 days.

On day 31 of the study the volunteers had the same regimen, namely reported to the hospital at 11:45 had lunch and tested at 12:00 and again at 13:00. The only difference on this day (day 31) was that the volunteers did not drink *composition 3* after lunch.

The Volunteers were examined by a battery of tests consisted of measurement of vital signs, blood pressure, heart rate and validated commonly used standard function and vigilance tests such as an immediate word recall test (short term memory), digit symbol substitution test (concentration), and subjective rating (on a visual analogue scale - VAS) of their vigilance, ability to focus, and effectiveness at work.

In each visit (day 1, day 30 and day 31) the results of the tests conducted at 12:00 and at 13:00 were compared to each other in order to evaluate the effect of drinking *composition 3.*

In addition the results of the tests on the three days of visit (day 1, day 30, day 31) were compared to each other, utilizing either paired t-test or one way analysis of variance, with p<0.05 considered statistically significant.

### Results:

The study was approved by the institutional review board (IRB, Helsinki committee) of Rambam Medical Center and all participants have signed an informed consent prior to participation.

Overall, ninety-five volunteers (40 males and 55 females) participated in this study. Their average age was 37±11 years (range 19-63 years), and their average BMI was 24.5 ± 1.7 Kg/m² (range 19.7-34.7).

The results of their functional tests before and following the drink on day 1, day 30, and day 31 are presented in Table 6. As detailed above, the volunteers did not drink *composition 3* on day 31.

As can be seen, in the first day drinking composition 3 resulted in an overall improvement in all the tested parameters. An objective improvement (iWRT and DSST) of about 8.5% and a subjective improvement (Vigilance, focusing and effectiveness) of about 14% were observed.

On day 30, drinking composition 3 resulted in an overall objective improvement of 7.3% and subjective improvement of about 11.5%.

On day 31, at which the volunteers only reported to the hospital but did not drink composition 3, the results of the objective tests deteriorated by 7.1%, and the subjective VAS measures deteriorated by about 11.7%.

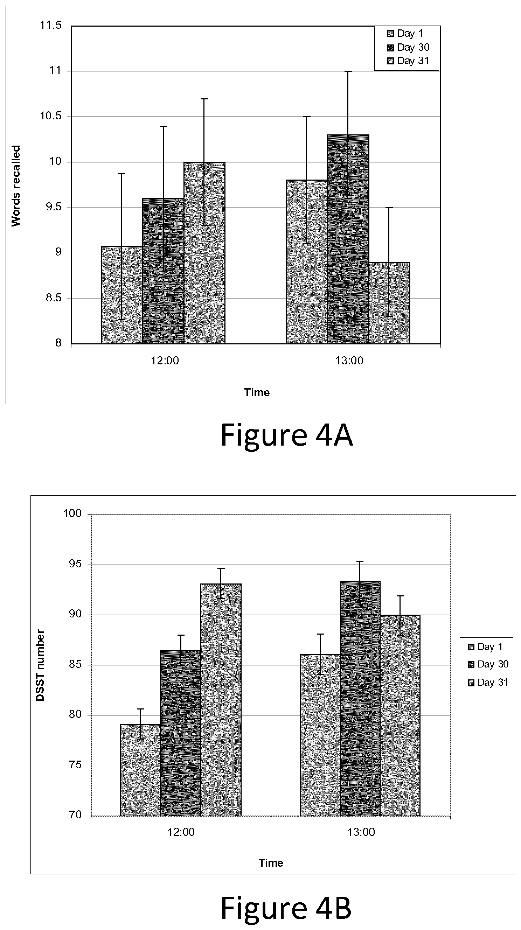

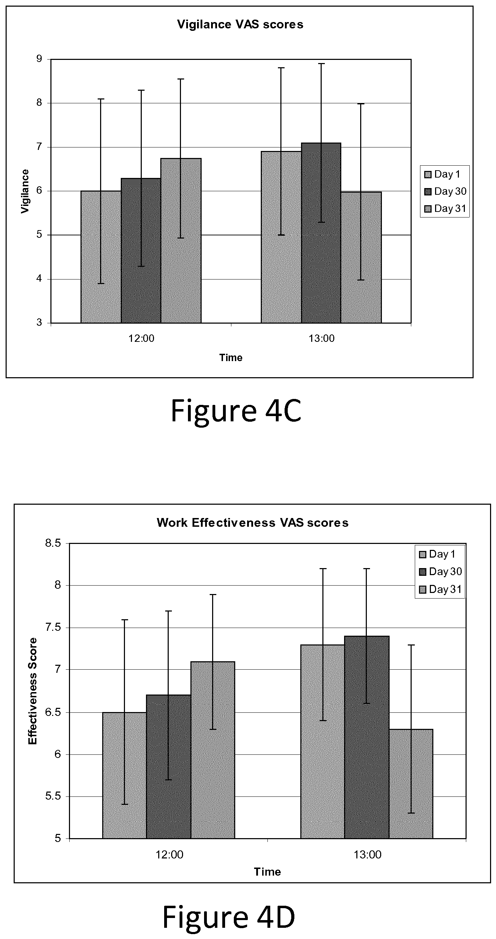

Interestingly, a gradual baseline improvement in the results of the various tests was observed (Table 6, Figure 4).

Word recalled at baseline increased from day 1 to day 30 by 5.5% and further increased in day 31 by additional 4.2 percent. Similarly DSST score increased from a baseline of 79 in day 1 to 87 in day 30 (rise of 10%) and to 93 in day 31 (additional rise of 6.9%). There was a similar trend in the VAS scores for vigilance, focusing and effectiveness (rises of 5-7% from the baseline of day 1 to the baseline of day 30, and a further rise of 6-7.5% to day 31). These data are presented in figures 1-4 (left panel, at 12:00) and in Table 6.

The hemodynamic measures at the various times are presented in Table 7. As can be seen, drinking composition 3 did not result in any significant change in blood pressure or pulse rate. The reduction of 0.4-2.5% in pulse rate and blood pressure following drinking composition 3 probably reflects the post lunch dip phenomenon, as is also seen on Day 31.

This clinical current study shows that without drinking composition 3, a post lunch dip phenomenon is observed on Day 31. Namely there is deterioration in the tested parameters after lunch. This is indicted by the reduction in the objective performance tasks by 7.1% and subjective alertness, focusing ability and work effectiveness by 11.2-11.9%. Pulse and blood pressure dropped by an average of 2.4%.

However, drinking composition 3 immediately after lunch these drops are not only blocked, but there an improvement is observed in both objective test performances and in subjective assessment of alertness, focusing ability and work effectiveness. The magnitude of these improvements ranged on day 1 between 8-14.9 percent.

Thus, given the expected drop in these parameters due to post lunch dip, the net effect of the drinking composition 3 seems to be close to 20%. Furthermore, this study demonstrates that a daily use of the composition over 30 days (one bottle of 100cc per day) does not result in tolerance or habituation, since the improvements observed in day 30 are similar to those observed in day 1, and range between 6.6-12.8 percent.

Importantly, these effects throughout the entire study did not adversely affect hemodynamic measures.

Finally, surprisingly, even baseline scores before drinking improved between Day 1 and Day 30 (by about 8%), and further improved on Day 31 (additional improvement of about 5%).

Without being bound by theory, it may be suggested that this may be associated with accumulative additive long term effect of the composition. This hypothesis is supported by the fact that subjective VAS is probably not associated with a learning curve.

**Table 6: Effect of daily administration of Composition 3 on physicomotorcognitive and behavioral tests**

| | **Day 1** | | | **Day 30** | | | **Day 31** | | |
|---|---|---|---|---|---|---|---|---|---|
| | Before WU | 1h after WU | Change (%) | Before WU | 1h after WU | Change (%) | After Lunch | 1h after Lunch | Change (%) |
| iWRT | 9.1±3.7 | 9.8±3.4 | +8.0 | 9.6±3.9 | 10.3±3.9 | +6.6 | 10.0±3.8 | 8.9±3.3 | -10.8 |
| DSST | 79±14 | 86±15 | +8.9 | 87±15 | 93±16 | +8.0 | 93±17 | 90±19 | -3.5 |
| Vigilance | 5.9±2.1 | 6.9±1.9 | +14.9 | 6.3±2.0 | 7.911.8 | +12.8 | 6.7±1.8 | 6.0±2.0 | -11.2 |
| Focusing | 6.0±1.9 | 6.9±1.8 | +14.8 | 6.3±2.0 | 7.1±1.9 | +11.4 | 6.8±1.7 | 6.0±2.0 | -11.9 |
| Effectiveness | 6.4±1.9 | 7.3±1.3 | +12.5 | 6.7±2.0 | 7.4±1.8 | +10.4 | 7.1±1.6 | 6.3±2.0 | -11.9 |

| | | | | | | | | | |
|---|---|---|---|---|---|---|---|---|---|
| iWRT = immediate Word Recall Test; DSST = Digit Symbol Substitution Test. WU = WakeUp® composition 3. | | | | | | | | | |

**Table 7: Effect of daily administration of Composition 3 on heart activity**

| | **Day 1** | | | **Day 30** | | | **Day 31** | | |
|---|---|---|---|---|---|---|---|---|---|
| | Before WU | 1h after WU | Change (%) | Before WU | 1h after WU | Change (%) | After Lunch | 1h after Lunch | Change (%) |
| Pulse | 74±11 | 73±10 | -0.4 | 77±10 | 75±11 | -2.5 | 76±11 | 73±11 | -3.5 |
| Systolic BP | 122±12 | 120±13 | -1.9 | 121±13 | 119±12 | -1.5 | 119±18 | 118±14 | -0.2 |
| Diastolic BP | 76±10 | 74±9 | -2.5 | 74±9 | 73±9 | -1.7 | 75±9 | 72±9 | -3.5 |

| | | | | | | | | | |
|---|---|---|---|---|---|---|---|---|---|
| WU = WakeUp® composition 3. | | | | | | | | | |

## Claims

1. A composition comprising
(i) between 30mg to 70mg of an extract of *guarana* plant or an isolate of *guarana* plant obtained by other isolation methods (not extraction) per 100ml of composition,
(ii) between 30mg to about 70mg of an extract of *ginkgo biloba* plant or an isolate of *ginkgo biloba* plant obtained by other isolation methods (not extraction) per 100ml of composition and
(iii) between 4ml fruit sugars to 15ml fruit sugars per 100ml of composition,
for use in ameliorating or inhibiting the temporary reduction of physical and/or mental functionality of a subject who is experiencing post lunch dip,
wherein the composition is administered at least once a day for a period of at least 7 days, and
wherein the composition exhibits a medically negligible or no detected effect on the subject's heart activity following administration.

2. The composition for use of claim 1, wherein the subject's heart activity is determined by one or more parameters selected from the group consisting of blood pressure, heart rate and pulse rate.

3. The composition for use of claim 1 or 2, wherein the medically negligible or no detected effect is determined within a time window of up to 24 hours post administration of said composition.

4. The composition for use of any one of claims 1 to 3, for administration once or twice a day.

5. The composition for use of any one of claims 1 to 4, for use for a period of at least fourteen days, preferably, in a period of at least thirty days.

6. The composition for use of any one of claims 1 to 5, for use during or before the subject experiences post lunch dip.

7. The composition for use of any one of claims 1 to 6, for use before noon or within a time window of up to an hour before post lunch is expected to develop in the subject.

8. The composition for use of any one of claims 1 to 7, comprising between 6mg *guaranine* to 20mg *guaranine,* between 6mg *flavonoid glycosides* to 20mg *flavonoid glycosides* ; and between 6ml fruit sugars to 10ml fruit sugars.

9. The composition for use of any one of Claims 1 to 8, comprising elderberry.

10. The composition for use of Claim 9, comprising between 40mg to 60mg elderberry per 100ml of composition.

11. The composition for use of any one of the preceding claims, wherein the composition is administered to a patient suffering from a pre-existing heart condition or a condition that would be aggravated by tachycardia .

12. The composition for use of any one of the preceding claims, wherein the composition is administered to a patient who is receiving one or more further therapeutic agents which provoke or aggravate tachycardia or hypertension.

## Patentansprüche

1. Zusammensetzung, die umfasst:
(i) zwischen 30 mg und 70 mg eines Extrakts der *Guaraná*-Pflanze oder eines Isolats der *Guaraná*-Pflanze, das durch andere Isolationsverfahren (nicht Extraktion) erhalten wurde, pro 100 ml Zusammensetzung,
(ii) zwischen 30 mg und ungefähr 70 mg eines Extrakts der *Ginkgo-biloba*-Pflanze oder eines Isolats der *Ginkgo-biloba*-Pflanze, das durch andere Isolationsverfahren (nicht Extraktion) erhalten wurde, pro 100 ml Zusammensetzung, und
(iii) zwischen 4 ml Fruchtzucker und 15 ml Fruchtzucker pro 100 ml Zusammensetzung
zur Verwendung bei der Verbesserung oder Hemmung der temporären Verringerung physischer und/oder physischer Funktionalität eines Individuums mit postprandialer Müdigkeit,
wobei die Zusammensetzung für einen Zeitraum von zumindest 7 Tagen zumindest einmal täglich verabreicht wird, und
wobei die Zusammensetzung nach Verabreichung eine medizinisch vernachlässigbare oder keine nachgewiesene Wirkung auf die Herzaktivität des Individuums hat.

2. Zusammensetzung zur Verwendung nach Anspruch 1, wobei die Herzaktivität des Individuums anhand eines oder mehrerer Parameter bestimmt wird, die aus der Gruppe ausgewählt sind, bestehend aus Blutdruck, Herzfrequenz und Pulsfrequenz.

3. Zusammensetzung zur Verwendung nach Anspruch 1 oder 2, wobei die medizinisch vernachlässigbare oder keine nachgewiesene Wirkung innerhalb eines Zeitfensters von bis zu 24 Stunden nach Verabreichung der Zusammensetzung bestimmt wird.

4. Zusammensetzung zur Verwendung nach einem der Ansprüche 1 bis 3 zur einmal oder zweimal täglichen Verabreichung.

5. Zusammensetzung zur Verwendung nach einem der Ansprüche 1 bis 4 zur Verwendung für einen Zeitraum von zumindest 14 Tagen, vorzugsweise in einem Zeitraum von zumindest 30 Tagen.

6. Zusammensetzung zur Verwendung nach einem der Ansprüche 1 bis 5 zur Verwendung während oder bevor das Individuum postprandiale Müdigkeit erleidet.

7. Zusammensetzung zur Verwendung nach einem der Ansprüche 1 bis 6 zur Verwendung vor Mittag oder innerhalb eines Zeitfensters bis zu einer Stunde vor erwartetem Auftreten einer postprandialen Müdigkeit beim Individuum.

8. Zusammensetzung zur Verwendung nach einem der Ansprüche 1 bis 7, die zwischen 6 mg *Guaranin* und 20 mg *Guaranin,* zwischen 6 mg *Flavonoidglykosiden* und 20 mg *Flavonoidglykosiden;* und zwischen 6 ml Fruchtzucker und 10 ml Fruchtzucker umfasst.

9. Zusammensetzung zur Verwendung nach einem der Ansprüche 1 bis 8, die Holunderbeere umfasst.

10. Zusammensetzung zur Verwendung nach Anspruch 9, die zwischen 40 mg und 60 mg Holunderbeere pro 100 ml Zusammensetzung umfasst.

11. Zusammensetzung zur Verwendung nach einem der vorstehenden Ansprüche, wobei die Zusammensetzung einem Patienten verabreicht wird, der an einem bereits bestehenden Herzleiden oder einem Leiden, das durch Tachykardie verschlimmert werden würde, leidet.

12. Zusammensetzung zur Verwendung nach einem der vorstehenden Ansprüche, wobei die Zusammensetzung einem Patienten verabreicht wird, der ein oder mehrere weitere therapeutische Mittel einnimmt, die Tachykardie oder Bluthochdruck hervorrufen oder verschlimmern.

## Revendications

1. Composition comprenant
(i) entre 30 mg et 70 mg d'un extrait de plante guarana ou d'un isolat de plante de guarana obtenu par d'autres procédés d'isolement (hors extraction) pour 100 ml de composition,
(ii) entre 30 mg et 70 mg d'un extrait de plante de *ginkgo biloba* ou d'un isolat de plant de *ginkgo biloba* obtenu par d'autres procédés d'isolement (hors extraction) pour 100 ml de composition et
(iii) entre 4 ml de sucres de fruits à 15 ml de sucres de fruits pour 100 ml de composition,
pour une utilisation dans l'amélioration ou l'inhibition de la réduction temporaire de fonctionnalité physique et/ou mentale chez un sujet subissant un creux d'après déjeuner,
la composition étant administrée au moins une fois par jour pendant une période d'au moins 7 jours, et
la composition présentant un effet médicalement négligeable ou non détecté sur l'activité cardiaque du sujet à la suite de l'administration.

2. Composition destinée à une utilisation de la revendication 1, dans laquelle l'activité cardiaque du sujet est déterminée par au moins un paramètre choisi dans le groupe constitué par la tension artérielle, le rythme cardiaque et le pouls.

3. Composition destinée à une utilisation de la revendication 1 ou 2, dans laquelle l'effet médicalement négligeable ou non détecté est déterminé dans une fenêtre temporelle allant jusqu'à 24 heures après l'administration de ladite composition.

4. Composition destinée à une utilisation de l'une quelconque des revendications 1 à 3, destinée à une administration une fois ou deux fois par jour.

5. Composition destinée à une utilisation de l'une quelconque des revendications 1 à 4, destinée à une utilisation pendant une période d'au moins quatorze jours, de préférence, une période d'au moins trente jours.

6. Composition destinée à une utilisation de l'une quelconque des revendications 1 à 5, destinée à une utilisation pendant que le sujet subit un creux d'après déjeuner ou avant celui-ci.

7. Composition destinée à une utilisation de l'une quelconque des revendications 1 à 6, destinée à une utilisation avant midi ou dans une fenêtre temporelle allant jusqu'à une heure avant l'horaire estimé de la survenue du creux d'après déjeuner chez le sujet.

8. Composition destinée à une utilisation de l'une quelconque des revendications 1 à 7, comprenant entre 6 mg de *guaranine* à 20 mg de *guaranine,* entre 6 mg de *glycosides flavonoïdes* à 20 mg de *glycosides flavonoïdes* ; et entre 6 ml de sucres de fruits à 10 ml de sucres de fruits.

9. Composition destinée à une utilisation de l'une quelconque des revendications 1 à 8, comprenant de la baie de sureau.

10. Composition destinée à une utilisation de la revendication 9, comprenant entre 40 mg à 60 mg de baie de sureau pour 100 ml de composition.

11. Composition destinée à une utilisation de l'une quelconque des revendications précédentes, la composition étant administrée à un patient atteint d'une affection cardiaque préexistante ou d'une affection qui serait aggravée par une tachycardie.

12. Composition destinée à une utilisation de l'une quelconque des revendications précédentes, la composition étant administrée à un patient atteint qui reçoit au moins un agent thérapeutique supplémentaire qui provoque ou aggrave une tachycardie ou une hypertension.
